# EUROPÄISCHE PATENTSCHRIFT

(11) **EP 3 454 919 B1**
(45) Veröffentlichungstag und Bekanntmachung des Hinweises auf die Patenterteilung: **12.04.2023**
(21) Anmeldenummer: 17720521.8
(22) Anmeldetag: 04.05.2017
(51) Int. Cl.: A61M 1/00, F04B 9/04, F04B 35/01, F04B 45/047

(54) **MEMBRANVAKUUMPUMPE**
MEMBRANE VACUUM PUMP
POMPE SOUS VIDE À MEMBRANE

(30) Priorität: 11.05.2016 EP 16169148
(43) Veröffentlichungstag der Anmeldung: 20.03.2019
(73) Patentinhaber: Medela Holding AG, 6340 Baar (CH)
(72) Erfinder: MELZER, Martin, 6330 Cham (CH); HONEGGER, Adrian, 6004 Luzern (CH); BANNWART, Lukas, 6343 Rotkreuz (CH)
(74) Vertreter: Grünecker Patent- und Rechtsanwälte PartG mbB
(86) Internationale Anmeldenummer: PCT/EP2017/060639
(87) Internationale Veröffentlichungsnummer: WO 2017/194383

(56) Entgegenhaltungen:
- EP-A2- 0 286 792
- DE-A1- 2 608 664
- US-A- 5 776 098
- US-A1- 2013 061 744

## Beschreibung

### TECHNISCHES GEBIET

Die vorliegende Erfindung betrifft eine Membranvakuumpumpe. Sie betrifft insbesondere medizinische Saugpumpen, wie sie beispielsweise in Brustpumpeneinheiten zum Abpumpen menschlicher Muttermilch oder für Drainagepumpeinheiten, insbesondere für die Thoraxdrainage, die Wunddrainage oder die Absaugung von anderen Körperflüssigkeiten oder Körperfett eingesetzt werden.

### STAND DER TECHNIK

Medizinische Saugpumpen, insbesondere Brustpumpen und Drainagepumpen benötigen eine gewisse Motorengrösse, um genügend Leistung zu erbringen. Dies ist insbesondere bei der Gestaltung von im Gebrauch portablen Pumpen ein Nachteil, da die Elektromotoren wesentlich das Gewicht der Pumpeinheiten bestimmen und zudem entsprechend Platz benötigen. Derartige Saugpumpen sind zudem relativ laut und stören die Mütter und ihr Umfeld sowie die Patienten wie auch das medizinische Fachpersonal.

US 5 776 098 offenbart eine Brustpumpe mit einem Elektromotor, welcher eine Rotationsbewegung der Motorenachse mit Hilfe eines Exzenters in eine Linearbewegung einer Verbindungs- oder Antriebsstange umwandelt. Die Verbindungsstange ist mit einer Vakuummembran verbunden, welche in einer Vakuumkammer einen Unterdruck erzeugt. Diese Pumpe hat sich in der Praxis zwar bewährt. Nachteilig ist jedoch, wie oben erwähnt, dass sie einen leistungsstarken Motor benötigt, da der Drehmomentverlauf am Motor eine relativ grosse Spitze aufweist.

Eine weitere Brustpumpe, bei welcher die Vakuummembran gleichzeitig als Fördermittel für die abzupumpende Milch dient, ist in WO 2011/035447 offenbart.

WO 2002/17992 sowie US 8 535 284 offenbaren Drainagepumpen.

WO 93/03295 A1 und EP 0 456 387 A1 offenbaren peristaltische Pumpen zur Verabreichung eines Medikaments, welche Kurvenscheiben verwenden.

Des Weiteren sind im Stand der Technik Mehrgelenkgetriebe, insbesondere Viergelenkgetriebe, bekannt. Beispielsweise ist der Tschebyschow-Lambda Mechanismus ein viergliedriges Koppelgetriebe, welches eine Drehbewegung in eine annähernd geradlinige Bewegung mit in einer Bewegungsrichtung nahezu konstanter Geschwindigkeit umwandelt.

DE 26 08 664 A1 offenbart eine Vorrichtung zur Erzeugung eines gleichförmigen Förderstroms mit zwei Kolbenpumpen und einer Kurvenscheibe. Der Saughub ist mittels einer Rückstellfeder gewährleistet. Es ist erwähnt, dass sich anstelle von Kolbenpumpen auch Membranpumpen verwenden lassen.

EP 0 286 792 A2 beschreibt eine Flüssigkeitsdosierpumpe mit einem motorisch angetriebenen Exzenter und einem durch den Exzenter entgegen der Kraft einer Rückholfeder verschiebbaren Pumpenstössel. Zwischen der Motorwelle und dem Exzenter ist ein Getriebe mit variablem Unter- und Übersetzungsverhältnis in Abhängigkeit vom Drehwinkel der Motorwelle angeordnet.

### DARSTELLUNG DER ERFINDUNG

Es ist deshalb eine Aufgabe der Erfindung, eine Membranvakuumpumpe zu schaffen, welche klein, leicht und kostengünstig ausgebildet werden kann.

Diese Aufgabe löst eine Membranvakuumpumpe mit den Merkmalen des Anspruchs 1.

Die erfindungsgemässe Membranvakuumpumpe weist eine Pumpkammer, eine Vakuummembran zur Erzeugung eines Unterdrucks in der Pumpkammer, einen Elektromotor mit einer Motorachse, ein Getriebe und ein Verbindungselement auf. Das Getriebe setzt eine Rotationsbewegung der Motorachse in eine zyklische, annähernd lineare Vor- und Rückwärtsbewegung des Verbindungselements um, wodurch das Verbindungselement eine zyklische Bewegung der Vakuummembran bewirkt. Erfindungsgemäss ist die Rückwärtsbewegung des Verbindungselements pro Zyklus durch einen ersten Rotationswinkel der Motorachse bewirkt, welcher aufgrund des Getriebes ungleich gross als ein zweiter Rotationswinkel der Motorachse ist, wobei der zweite Rotationswinkel die Vorwärtsbewegung des Verbindungselements pro Zyklus bewirkt.

Diese Membranvakuumpumpe eignet sich vorzugsweise als medizinische Saugpumpe, insbesondere als Aggregat in einer Brustpumpeneinheit oder in einer Drainagepumpeneinheit. Sie eignet sich insbesondere für den Einsatz in Pumpeinheiten, welche batteriebetrieben sind.

Für die eine Bewegungsrichtung wird ein Bereich von mehr als 180° der Rotationsbewegung der Motorenachse, d.h. der Antriebswelle, verwendet. Dadurch lässt sich das Lastmoment, d.h. das Drehmoment am Motor, für diese Bewegungsrichtung auf einen relativ grossen Winkelbereich verteilen. Drehmomentspitzen und somit auch Lastspitzen lassen sich somit reduzieren. Vorzugsweise ist diese Bewegungsrichtung die Saughubrichtung. Die Saughubrichtung, in welcher die Membran aus ihrer Ausgangsposition und von der Wand der Pumpkammer weggezogen wird, wird hier als Rückwärtsrichtung bezeichnet. "Vorne" ist gemäss Text somit der Bereich der Pumpkammer, "hinten" ist der Bereich des der Pumpmembran beabstandeten Endes des Verbindungselements.

Erfindungsgemäss lassen sich somit kleinere und leistungsschwächere, jedoch auch geräuschärmere Motoren als beim eingangs erwähnten exzentrischen Kurbelgetriebe verwenden.

Wird hingegen derselbe Motorentyp wie bei einem exzentrischen Kurbelgetriebe verwendet, so ist die Effizienz bzw. die Leistung gesteigert.

Somit lässt sich durch geeignete Wahl des Motors ein Optimum zwischen Grösse, Leistung und Lautstärke der Pumpe finden.

Vorteilhaft ist ferner, dass die Membran durch eine lineare oder eine zumindest annähernd lineare Bewegung betrieben ist. Dies schont die Membran und minimiert ihren Verschleiss.

Nicht nur die Verwendung leistungsschwächerer Motoren reduziert die Geräuschbelastung, sondern auch die Verlangsamung der Membranbewegung in Saughubrichtung, welche durch den längeren Weg des Verbindungselements in diese Saughubrichtung bedingt ist. Dank dieser Verlangsamung der Membranbewegung ist die Pulsation des Fördermediums, üblicherweise Luft, reduziert.

Üblicherweise entspricht ein Bewegungszyklus des Verbindungselements und der Vakuummembran einer Rotationsbewegung der Motorachse um einen Gesamtwinkel von 360°.

Erfindungsgemäß definiert die Rückwärtsbewegung des Verbindungselements den Pumpenhub, welcher in der Vakuumkammer das Vakuum erzeugt, wobei der dieser Rückwärtsbewegung zugehörige erste Rotationswinkel grösser als der zweite Rotationswinkel ist. Dadurch lässt sich die Last über einen grösseren Zeitraum verteilen und die Lastspitze ist minimiert.

Vorzugsweise ist der erste Rotationswinkel grösser als 180°, wobei er vorzugsweise 200° bis 300° beträgt und noch bevorzugter 240° bis 270° beträgt. Vorzugsweise wird 2/3 bis zu 3/4 der Umdrehung der Motorachse für die Rückwärtsbewegung bzw. die Pumpenhubbewegung verwendet. Diese Pumpenhubbewegung erfolgt deshalb vorzugsweise um einiges langsamer als die Gegenbewegung.

Vorzugsweise ist der zweite Rotationswinkel kleiner als 180°, wobei er vorzugsweise 60° bis 160° beträgt und noch bevorzugter 90° bis 120° beträgt. Vorzugsweise wird lediglich 1/3 bis zu 1/4 der Umdrehung der Motorachse für die Gegenbewegung des Saughubs verwendet. Diese Gegenbewegung erfolgt entsprechend schneller als die Saughubbewegung.

Vorzugsweise ist das Drehmoment an der Motorachse für die Rückwärtsbewegung des Verbindungselements über einen Winkel von grösser als 180° verteilt. Dies verteilt das Drehmoment am Motor über einen grösseren Bereich. Grosse Drehmomentspitzen werden vermieden.

Die oben genannten Merkmale lassen sich mit unterschiedlichen Getrieben erhalten. In einer bevorzugten Ausführungsform ist das Getriebe ein Kurvenscheibengetriebe, welche eine von einer Kreisform abweichende und im Betrieb der Membranvakuumpumpe rotierende Kurvenscheibe aufweist. Je nach Form der Kurvenscheibe lässt sich ein während der Rückwärtsbewegung annähernd konstant bleibendes Drehmoment am Motor erzielen. Die Bewegung des Verbindungselements lässt sich linear ausführen.

Vorzugsweise ist das Verbindungselement in diesem Fall eine Verbindungsstange, welche mit einem ersten Ende mit der Vakuummembran verbunden ist und welche mit einem zweiten Ende an der Kurvenscheibe gelagert ist. Es ist ferner eine Führung vorhanden, welche die Verbindungsstange in die annähernd lineare, vorzugsweise genau lineare Bewegung führt.

Die Kurvenscheibe lässt sich aus Metall, einem beschichteten Metall, einem Keramik-Kunststoffverbund, einer beschichteten Keramik, einem Metall-Kunststoffverbund oder aus anderen Materialien ausführen. Vorzugsweise ist sie aus Kunststoff, insbesondere aus einem Polymer, gefertigt. Zudem ist das Getriebe einfach und kostengünstig herstellbar und mit geringem Gewicht ausführbar.

In einer anderen Ausführungsform ist das Getriebe ein Mehrgelenkgetriebe, vorzugsweise ein Viergelenkgetriebe. Vorzugsweise ist es als Tschebyschow-Lambda Mechanismus ausgebildet. Vorzugsweise bildet das Verbindungselement ein Gelenkglied des Getriebes. Vorzugsweise wird das Verbindungselement dabei auf einer annähernd geraden Linie in Saughubrichtung, d.h. in Rückwärtsrichtung, mit konstanter Geschwindigkeit bewegt. In Gegenrichtung erfolgt die Bewegung schneller.

Das Mehrgelenkgetriebe kann unterschiedlich ausgeführt sein. Es kann beispielsweise mehrere schwenkbar relativ zueinander gehaltene und ansonsten voneinander unabhängige Gelenkglieder aufweisen. Die Gelenke, welche die Schwenkachsen definieren, sind in diesem Fall vorzugsweise Wälz- oder Kugellager.

Mehrere Gelenkglieder oder alle Gelenkglieder können jedoch auch gemeinsam einstückig ausgebildet sein. In diesem Fall sind die Schwenkachsen vorzugsweise als Filmscharniere, vorzugsweise durch Materialverdünnung, ausgebildet. Vorzugsweise ist das Getriebe gemeinsam mit dem Verbindungselement einstückig ausgebildet. Eine derartige einstückige Ausbildung benötigt relativ wenig Platz und Material und ist zudem in der Herstellung kostengünstig. Das Mehrgelenkgetriebe kann aus denselben Materialen wie bereits oben erwähnt ausgebildet sein. Vorzugsweise ist es aus Metall oder Kunststoff oder einer Kombination davon ausgebildet. Die einstückigen Ausführungsformen sind vorzugsweise aus Kunststoff, insbesondere aus einem Polymer, gefertigt.

Die erfindungsgemässe Membranvakuumpumpe ermöglicht eine relativ gleichmässige und reduzierte Belastung des Elektromotors, einen vibrations- und geräuscharmen Betrieb sowie eine kostengünstige und kompakte Bauweise. Der Wirkungsgrad lässt sich zudem erhöhen. Derartige Membranvakuumpumpen lassen sich in allen Grössen ausführen. Besonders geeignet sind sie für relativ kleine, auch im Gebrauch portabel ausgebildete, batteriebetriebene Saugpumpeneinheiten, wie sie beispielsweise eingangs erwähnt sind.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung einer erfindungsgemässen Membranvakuumpumpe in einer ersten Ausführungsform;
- Figur 2: einen ersten Längsschnitt durch die Pumpe gemäss Figur 1;
- Figur 3: einen zweiten Längsschnitt durch die Pumpe gemäss Figur 1;
- Figur 4: eine perspektivische Darstellung einer erfindungsgemässen Membranvakuumpumpe in einer zweiten Ausführungsform;
- Figur 5: einen Querschnitt durch die Pumpe gemäss Figur 4;
- Figur 6: einen Längsschnitt durch die Pumpe gemäss Figur 4;
- Figur 7: eine perspektivische Darstellung des Getriebes mit Motor und Verbindungselement gemäss Figur 4;
- Figur 8: eine erste Seitenansicht der Einheit gemäss Figur 7;
- Figur 9: eine Ansicht der Einheit gemäss Figur 7 von hinten;
- Figur 10: eine zweite Seitenansicht der Einheit gemäss Figur 7;
- Figur 11: eine perspektivische Darstellung einer erfindungsgemässen Membranvakuumpumpe in einer dritten Ausführungsform;
- Figur 12: einen Querschnitt durch die Pumpe gemäss Figur 11;
- Figur 13: einen Längsschnitt durch die Pumpe gemäss Figur 11;
- Figur 14: eine perspektivische Darstellung des Getriebes mit Motor und Verbindungselement gemäss Figur 11;
- Figur 15: eine erste Seitenansicht der Einheit gemäss Figur 11;
- Figur 16: eine Ansicht der Einheit gemäss Figur 11 von hinten;
- Figur 17: eine zweite Seitenansicht der Einheit gemäss Figur 11;
- Figur 18: ein Diagramm, welches das relative Drehmoment am Motor in Funktion des Rotationswinkels der Motorachse zeigt und
- Figur 19: ein Diagramm, welches die Position des Verbindungselements in Funktion des Rotationswinkels der Motorachse zeigt.

Gleiche Teile sind mit gleichen Bezugszeichen versehen.

### BESCHREIBUNG BEVORZUGTER AUSFÜHRUNGSFORMEN

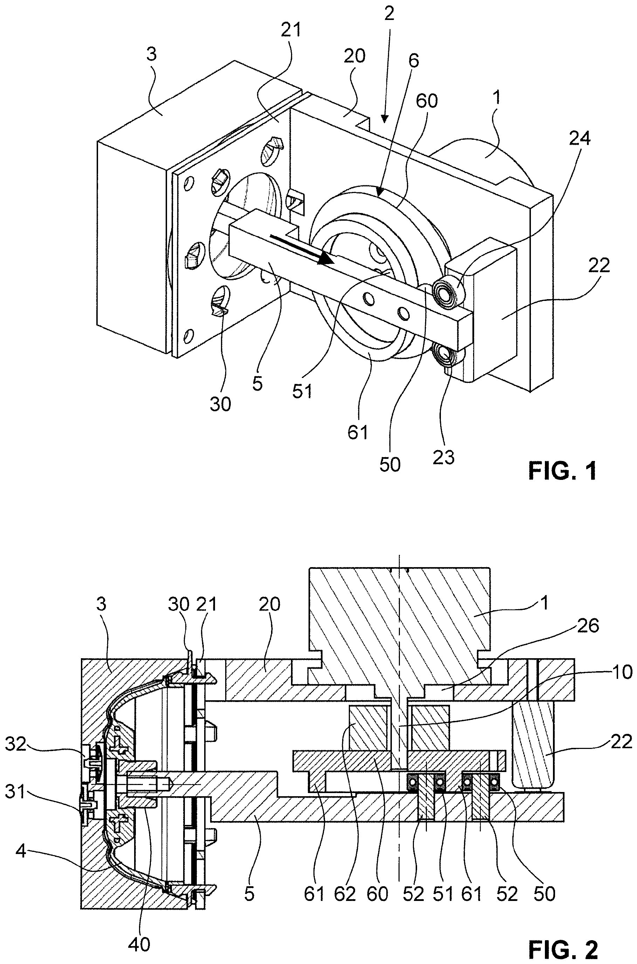

In den Figuren 1 bis 3 ist ein erstes Ausführungsbeispiel der erfindungsgemässen Membranvakuumpumpe dargestellt. Diese weist einen Elektromotor 1 auf. Dieser Elektromotor 1 ist in einer Halterung 2 mit einer Grundplatte 20 gehalten. Eine Elektronik zur Steuerung des Motors, ein Gehäuse zur Aufnahme der dargestellten Elemente sowie Bedienungsmittel wie Schalter und Drehknöpfe zur Betätigung der Pumpe sind nicht dargestellt. Die Form und Ausbildung der Halterung 2 ist lediglich beispielhaft zu verstehen. Andere Formen und Arten der Befestigung der einzelnen Elemente der Pumpe, insbesondere des Motors sowie der nachfolgend beschriebenen Pumpkammer 3 und Verbindungselement 5 sind möglich.

Der Elektromotor 1 weist eine Antriebswelle, hier Motorachse 10 genannt, auf. Die Motorachse 10 durchdringt unbehindert eine Durchgangsöffnung in der Grundplatte 20. Die Motorachse 10 ist mit einem Kurvenscheibengetriebe 6 fest verbunden. Das Getriebe 6 weist ein Kupplungselement 62 auf, welches das restliche Getriebe mit der Motorachse 10 verbindet. Die Motorachse 10 durchdringt die Grundplatte 20 beabstandet und unbehindert. Das freie Ende der Motorachse ist fest mit der ebenfalls mit dem Kupplungselement 62 verbundenen Basisscheibe 60 verbunden, so dass sich diese gemeinsam mit dem Kupplungselement 62 mit der Motorachse 10 dreht. Die Basisscheibe 60 ist vorzugsweise in ihrem Zentrum mit der Motorachse 10 verbunden. Die Befestigungslöcher für die Verbindung mit dem Kupplungselement 62 sind mit dem Bezugszeichen 27 versehen.

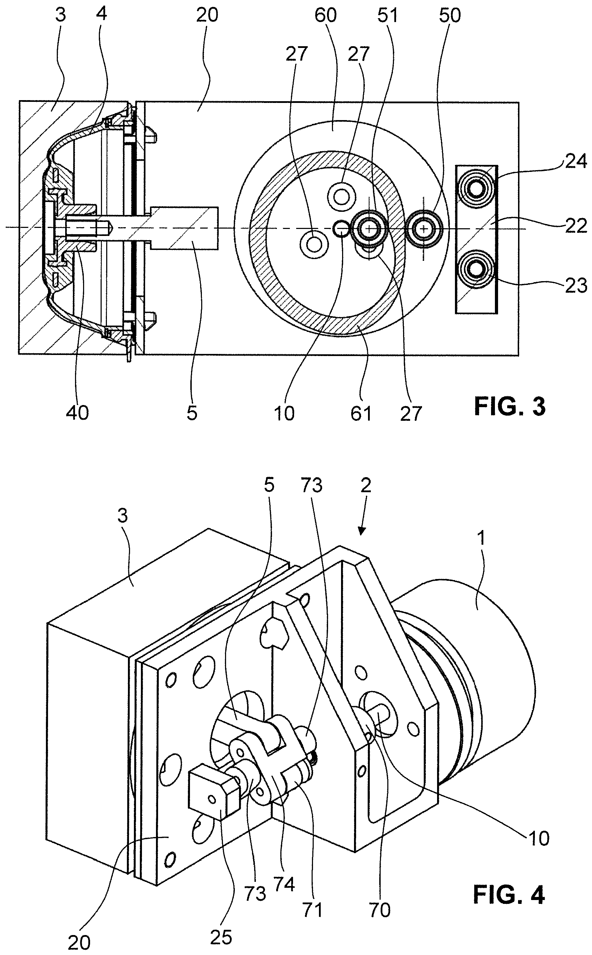

Auf der Basisscheibe 60 ist ein vorstehender Kurvenring 61 angeordnet. Basisscheibe 60 und Kurvenring 61 bilden gemeinsam die Kurvenscheibe. Der Kurvenring 61 kann auf der Basisscheibe 60 befestigt sein, also ein getrenntes Bauteil sein, oder einstückig mit der Basisscheibe 60 ausgebildet sein. Letzteres ist bevorzugt. Der Kurvenring 61 weist, wie in Figur 3 gut erkennbar ist, eine von einem runden Kreis abweichende Form auf. Zudem weist er vorzugsweise eine sich verändernde Wanddicke auf, wie dies ebenfalls in Figur 3 gut erkennbar ist. Die Form entspricht im breitesten Sinne einem Spiralsegmente beschreibenden Polynom. Die Wanddicke des Kurvenrings 61 variiert so, dass nachfolgend beschriebene Mitnehmerrollen 50, 51 in jedem Rotationswinkel spielfrei anliegen.

Wie am besten in Figur 1 erkennbar ist, ist ein Verbindungselement 5 vorhanden, welches das Getriebe 6 und somit den Motor 1 mit einer Pump- oder Vakuummembran 4 verbindet. Die Vakuummembran 4 ist in einer Vakuumkammer 3 angeordnet, wobei die Vakuummembran 4 die bewegliche Rückwand der Vakuumkammer 3 bildet. Eine Abdeckplatte 21 der Halterung 2 ist ebenfalls in diesem rückseitigen Bereich befestigt. Die entsprechenden Fixierungselemente, hier Einrasthaken, weisen das Bezugszeichen 30 auf.

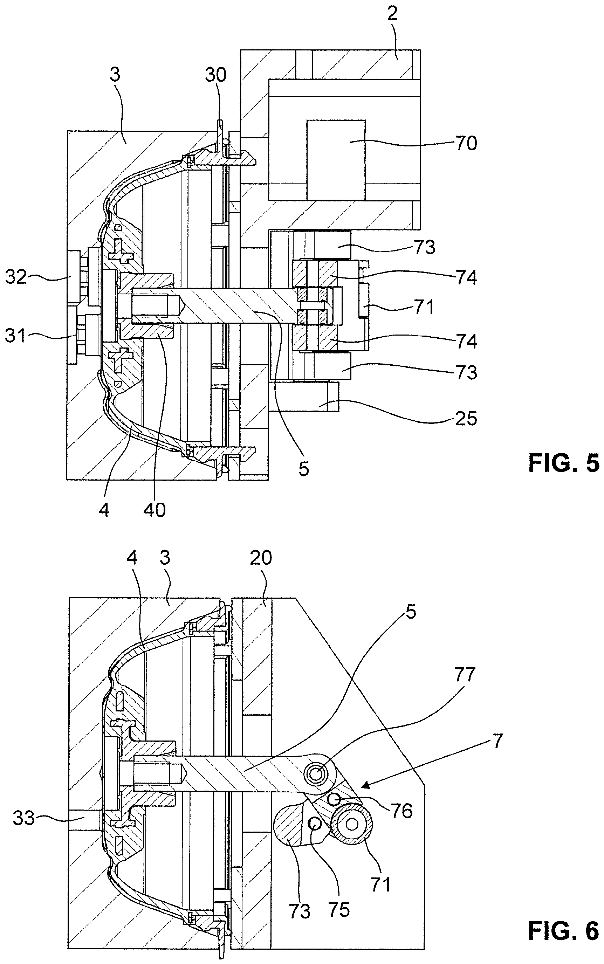

Die Pumpkammer 3 umfasst vorzugsweise zudem ein Einlassventil 32 sowie ein Auslassventil 31 zur Be- und Entlüftung der Vakuumkammer 3. Ein Vakuumanschluss zur Verbindung mit einer Saugleitung oder einer Saugkappe, z.B. einer Brusthaube, ist in den Figuren 1 bis 3 nicht sichtbar. In Figur 6 ist er mit dem Bezugszeichen 33 versehen. Diese Pumpkammer 3 mit Vakuummembran 4 weist die übliche Form auf und wird nachfolgend nicht näher beschrieben.

Die Vakuummembran 4 weist ein Kopplungselement 40 auf, beispielsweise eine zylinderförmige Aufnahme, in welcher ein Ende des Verbindungselements 5 fixiert gehalten ist.

Ein zweites Ende des stab- oder stangenförmigen Verbindungselements 5 ist in einer Linearführung 22 der Halterung 2 linear verschiebbar gehalten. Hierfür weist die Linearführung 22 ein Rollenpaar mit einer ersten Führungsrolle 23 und einer zweiten Führungsrolle 24 auf. Diese sind ober- und unterhalb des Verbindungselements 5 angeordnet und klemmen dieses ein, so dass das Verbindungselement 5 zwischen ihnen geführt hindurchgleitet.

Am Verbindungselement 5 sind eine erste und eine zweite Mitnehmerrolle 50, 51 befestigt, welche mit ihren Drehachsen zum Kurvenring 61 hingewandt sind und diesen zwischen sich lagern. Die Befestigung erfolgt beispielsweise mittels Befestigungsstifte 52, welche die Mitnehmerrollen 50, 51 drehbar an der Verbindungsstange 5 fixieren.

Bei Drehung der Motorachse 10 dreht sich somit die Basisscheibe 60 mit dem Kurvenring 61. Die Mitnehmerrollen 50, 51 rollen am Kurvenring 61 ab und treiben so die Verbindungsstange 5 an. Die Mitnehmerrollen 50, 51 führen dabei gemeinsam mit den Führungsrollen 23, 24 dazu, dass die Verbindungsstange 5 nach Vorgabe der Bewegung des Kurvenrings 61 in einer linearen Rückwärtsbewegung von der Pumpkammer 3 weg bewegt wird und die Vakuummembran 4 mit sich zieht. Die Rückwärtsbewegung ist in den Figuren mit einem Pfeil bezeichnet.

In der Pumpkammer 3 entsteht ein Unterdruck. Nach Massgabe der Form des Kurvenrings 61 bewegt sich die Verbindungsstange 5 wieder nach vorne, so dass die Vakuummembran 4 in ihre Ausgangsposition zurückfinden kann.

Durch die spezielle Form des Kurvenrings 61 benötigt die Verbindungsstange 5 länger für die Rückwärtsbewegung als für die Vorwärtsbewegung. Die Rückwärtsbewegung ist langsamer als die Bewegung in Gegenrichtung. Zudem benötigt sie einen grösseren Winkelbereich der Drehung der Motorachse 10 als die Vorwärtsbewegung.

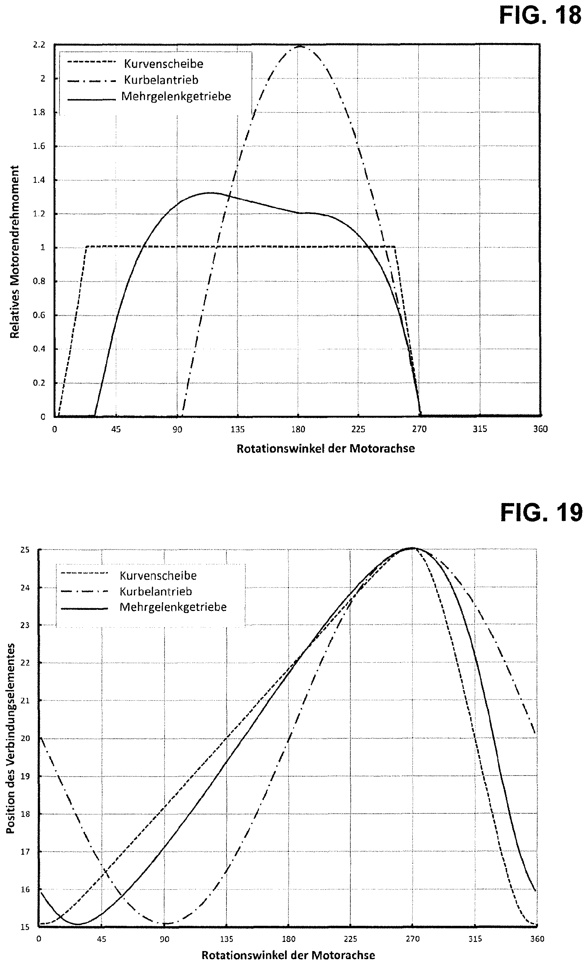

Wie in den Figuren 18 und 19 erkennbar ist, benötigt die Verbindungsstange 5 eine Rotationsbewegung der Motorachse 10 von 0° bis 270°, also 3/4 der gesamten Umdrehung pro Zyklus, um die maximale Saughubposition zu erreichen. Für die Vorwärtsbewegung der Verbindungsstange 5 in die Ausgangsposition der Vakuummembran 4 genügen hingegen 90°, also 1/4 der gesamten Umdrehung der Motorachse 10 pro Zyklus. Dies ist in Figur 19 zu sehen.

Das aufzuwendende Drehmoment bzw. das Lastmoment an der Motorachse 10 bzw. am Motor 1 ist in Figur 18 gezeigt. Wie dort erkennbar ist, steigt das Drehmoment bei Beginn des Zyklus sehr schnell an und verläuft dann über einen Winkelbereich von fast 270° konstant, bis es zum Schluss der Rückwärtsbewegung mit grosser negativer Steigung wieder absinkt.

Der Vergleich zum ebenfalls dargestellten Drehmoment eines Kurbelantriebs zeigt sich, dass die beim Kurbelantrieb vorhandene Drehmomentspitze, deren Quadrat der Spitzenleistung entspricht, nun massiv minimiert ist. Wie in den Figuren 18 und 19 erkennbar, sind die Vor- und Rückwärtsbewegungen beim exzentrischen Kurbelantrieb symmetrisch, so dass sie je einen Winkelbereich von 180° beanspruchen und die Antriebsstange in beiden Richtungen gleich schnell bewegt wird.

Ebenfalls in den Figuren 18 und 19 dargestellt, ist das Verhalten einer erfindungsgemässen Membranvakuumpumpe mit einem Mehrgelenkgetriebe. Zwei Varianten davon sind nachfolgend beschrieben. Wie in den Figuren 18 und 19 erkennbar ist, erstreckt sich auch hier die Rückwärtsbewegung des Verbindungselements, d.h. der Saughub, über einen Winkelbereich, welcher wesentlich grösser als 180° ist. Er ist in Figur 18 ca. 250°. Die Vorwärtsbewegung erfolgt schneller und erstreckt sich lediglich über einen Winkelbereich von ca. 110°. Die Bewegung des Verbindungselements 5 ist annähernd linear, muss aber nicht zwingend genau linear sein. In Figur 19 ist erkennbar, dass auch hier die Spitzenlasten und Drehmomentspitzen abgeschwächt sind. Das Drehmoment an der Motorachse 10 bzw. dem Motor 1 ist annähernd konstant, wobei es nicht so konstant ist wie bei Verwendung der Kurvenscheibe.

Eine erste Variante einer Membranvakuumpumpe mit einem Mehrgelenkgetriebe 7 ist in den Figuren 4 bis 10 dargestellt. Der Elektromotor 1 ist in der Halterung 2 gehalten, wobei die Motorachse 10 die Halterung 2 ungehindert durchsetzt. Die Motorachse 10 ist drehfest über ein Kupplungselement 70 mit einem Exzenterelement 71 verbunden. Die Rotationsachse der Motorachse ist in den Figuren mit dem Bezugszeichen R versehen.

Ein gabelförmiges erstes Gelenkglied 73 umgreift das Exzenterelement 71, ohne direkt mit diesem verbunden zu sein. Es ist an einem Ende an einem Lager 25 der Halterung 2 schwenkbar gelagert. Diese Lagerung bildet die erste Schwenkachse 75. Über eine zweite Schwenkachse 76 ist das erste Gelenkglied 73 mit einem zweiten Gelenkglied 74 schwenkbar verbunden. Das zweite Gelenkglied 74 ist zudem über eine dritte Schwenkachse 77 schwenkbar mit der Verbindungsstange 5 verbunden sowie über eine vierte Schwenkachse 78 mit dem Exzenterelement 71. Die Verbindungsstange 5 bildet ein drittes Gelenkglied.

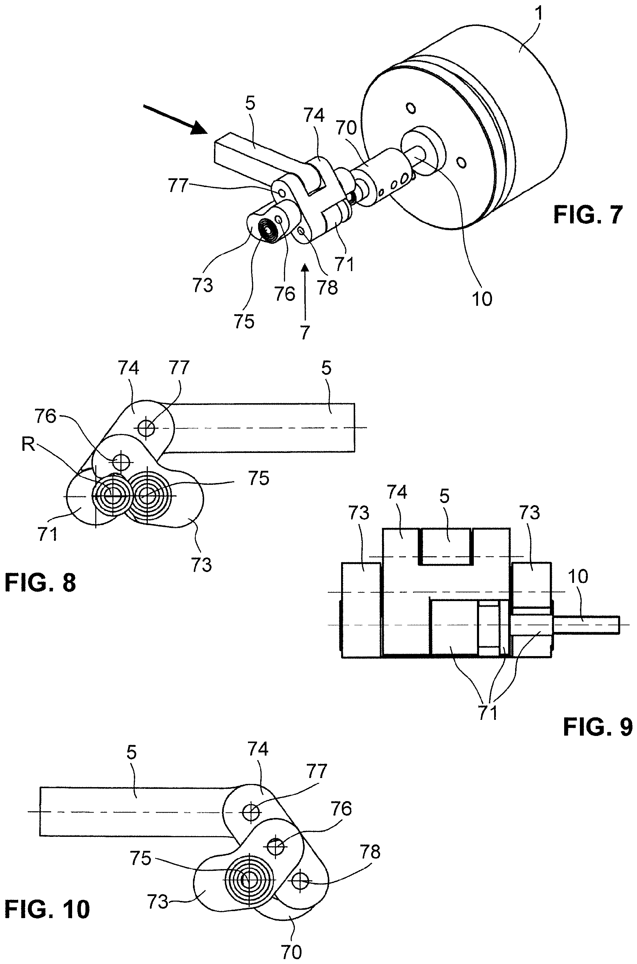

Diese Verbindungen sind in der Zusammenschau den Figuren 7 bis 10 gut erkennbar. Die Schwenkachsen sind in diesem Beispiel vorzugsweise durch Kugellager oder Wälzlager gebildet.

Diese Verbindungen sowie die Ausbildung und Anordnung der einzelnen Gelenkglieder führt dazu, dass die Rotationsbewegung in eine annähernd oder eine genau lineare Bewegung der Verbindungsstange 5 umgesetzt wird, wobei die Rückwärtsbewegung, d.h. die Saughubbewegung, zur Auslenkung der Vakuummembran 4 über einen grösseren Winkelbereich und langsamer erfolgt als die Vorwärtsbewegung. Die Positionen der Verbindungsstange 5 sowie das Drehmoment über dem Motor 1 sind wie erwähnt in den Figuren 18 und 19 dargestellt und wurden weiter oben im Text bereits beschrieben.

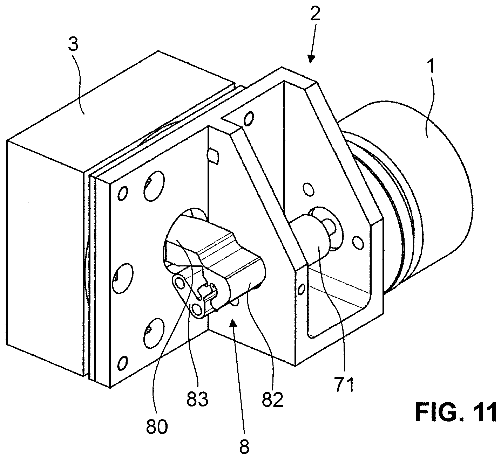

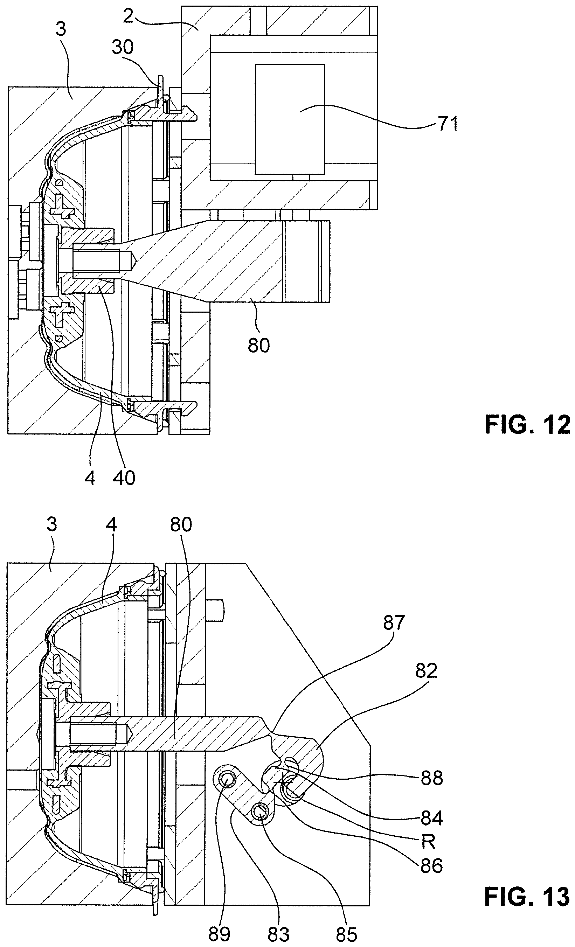

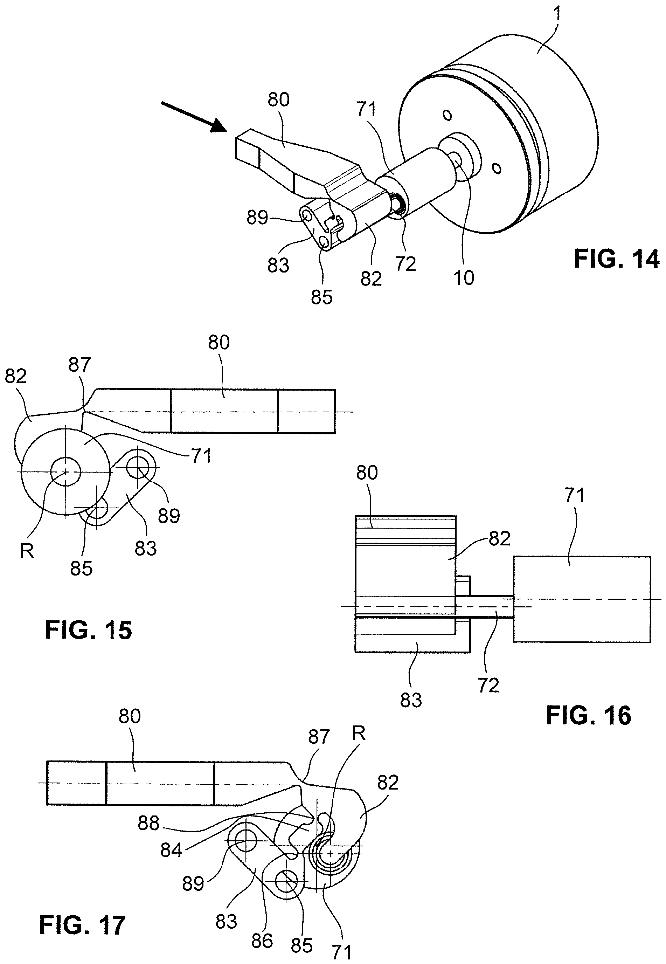

In der zweiten Variante des Mehrgelenkgetriebes gemäss den Figuren 11 bis 17 ist das Getriebe inklusive die Verbindungsstange einstückig ausgebildet. Es besteht vorzugsweise aus Kunststoff oder Metall. Motor 1, Motorachse 10, Pumpkammer 3, Halterung 2 und Exzenterelement 71 sind wie oben erwähnt ausgebildet. Hier passiert eine Exzenterachse 72 des Exzenterelements 71 die Halterung 2 ungehindert und dreht sich um die Rotationsachse R.

Das Getriebe ist als Filmscharniergelenkgetriebe 8 ausgebildet. Es weist einen stab- oder stangenförmigen Verbindungsarm 80 auf, welcher den vorgängig beschriebenen Verbindungsstab 5 ersetzt. Ferner umfasst das Getriebe 8 ein erstes Gelenkglied 82, ein zweites Gelenkglied 83, ein drittes Gelenkglied 84 sowie eine erste Schwenkachse 86, eine zweite Schwenkachse 87 sowie eine dritte Schwenkachse 88, welche die einzelnen Gelenkglieder sowie den Verbindungsarm, wie bereits anhand der ersten Variante beschrieben, miteinander verbinden. Die Bezugszeichen 85 und 89 zeigen einen ersten und einen zweiten Befestigungspunkt des Verbindungsarmes 80 mit der Halterung 2. Die erste, zweite und dritte Schwenkachse 86, 87, 88 sind als Filmscharniere ausgebildet, vorzugsweise durch Materialverdünnung. Das erste Gelenkglied 82 ist im Querschnitt vorzugsweise bogenförmig ausgebildet und geht über die zweite Schwenkachse 87 in den Verbindungsarm 80 über. Über die dritte Schwenkachse 88 geht es ferner in das dritte Gelenkglied 84 über. Das dritte Gelenkglied 84 ist im Querschnitt annähernd rechteckig ausgebildet, wobei die dritte Schwenkachse 88 an einer ersten Ecke davon angeordnet ist. An einer diametral gegenüberliegenden Ecke befindet sich die erste Schwenkachse 86, welche das dritte Gelenkglied 84 mit dem zweiten Gelenkglied 83 verbindet. Das zweite Gelenkglied 83 weist im Querschnitt eine abgerundete Rechteckform auf, ist jedoch um ein Vielfaches grösser ausgebildet als das dritte Gelenkglied 83 und weist annähernd dieselbe oder eine leicht grössere Querschnittfläche auf als das erste Gelenkglied 82. Der Verbindungsarm 80 bildet wiederum das vierte Gelenkglied des Getriebes 8.

Auch in dieser Variante führt die Rotationsbewegung der Motorachse 10 dazu, dass sich der Verbindungsarm 80 annähernd oder genau linear bewegt, wobei er in der Rückwärtsbewegung wie in der ersten Variante eine annähernd gleichmässige, aber geringe Geschwindigkeit aufweist und für den vollständigen Saughub einen grösseren Winkelbereich der Rotationsbewegung benötigt als die Vorwärtsbewegung. Die Informationen dazu sind wiederum in den Figuren 18 und 19 und der obigen Beschreibung zu finden.

Die erfindungsgemässe Membranvakuumpumpe lässt sich somit geräuscharm und trotzdem mit einer optimalen Leistung ausbilden.

**BEZUGSZEICHENLISTE**

| | | | |
|---|---|---|---|
| 1 | Elektromotor | 60 | Basisscheibe |
| 10 | Motorachse | 61 | Kurvenring |
| | | 62 | Kupplungselement |
| 2 | Halterung | | |
| 20 | Grundplatte | 7 | Mehrgelenkgetriebe |
| 21 | Abdeckplatte | 70 | Kupplungselement |
| 22 | Linearführung | 71 | Exzenterelement |
| 23 | erste Führungsrolle | 72 | Exzenterachse |
| 24 | zweite Führungsrolle | 73 | erstes Gelenkglied |
| 25 | Lager | 74 | zweites Gelenkglied |
| 26 | Durchgangsöffnung | 75 | erste Schwenkachse |
| 27 | Befestigungslöcher | 76 | zweite Schwenkachse |
| | | 77 | dritte Schwenkachse |
| 3 | Vakuumkammer | 78 | vierte Schwenkachse |
| 30 | Fixierungselement | | |
| 31 | Auslassventil | 8 | Filmscharniergelenkgetriebe |
| 32 | Einlassventil | 80 | Verbindungsarm |
| 33 | Drucksensoranschluss | 82 | erstes Gelenkglied |
| | | 83 | zweites Gelenkglied |
| 4 | Pumpmembran | 84 | drittes Gelenkglied |
| 40 | Kopplungselement | 85 | erster Befestigungspunkt |
| | | 86 | erste Schwenkachse |
| 5 | Verbindungsstange | 87 | zweite Schwenkachse |
| 50 | erste Mitnehmerrolle | 88 | dritte Schwenkachse |
| 51 | zweite Mitnehmerrolle | 89 | zweiter Befestigungspunkt |
| 52 | Befestigungsstift | | |
| | | R | Rotationsachse |
| 6 | Kurvenscheibengetriebe | | |

## Patentansprüche

1. Membranvakuumpumpe mit einer Pumpkammer (3), einer Vakuummembran (4) zur Erzeugung eines Unterdrucks in der Pumpkammer (3), einem Elektromotor (1) mit einer Motorachse (10), einem Getriebe (6, 7, 8) und einem Verbindungselement (5, 80),
wobei das Getriebe (6, 7, 8) eine Rotationsbewegung der Motorachse (10) in eine zyklische, annähernd lineare Vor- und Rückwärtsbewegung des Verbindungselements (5, 80) umsetzt, wodurch das Verbindungselement (5, 80) eine zyklische Bewegung der Vakuummembran (4) bewirkt,
wobei ein zweiter Rotationswinkel der Motorachse (10) die Vorwärtsbewegung des Verbindungselements (5, 80) pro Zyklus bewirkt,
**dadurch gekennzeichnet, dass**
die Rückwärtsbewegung des Verbindungselements (5, 80) pro Zyklus durch einen ersten Rotationswinkel der Motorachse (10) bewirkt ist, welcher aufgrund des Getriebes (6, 7, 8) ungleich gross als der zweite Rotationswinkel der Motorachse (10) ist,
und dass die Rückwärtsbewegung des Verbindungselements (5, 80) einen Pumpenhub des Zyklus definiert, welcher in der Vakuumkammer (3) das Vakuum erzeugt, und wobei der erste Rotationswinkel grösser als der zweite Rotationswinkel ist.

2. Membranvakuumpumpe nach Anspruch 1, wobei ein Bewegungszyklus des Verbindungselements (5, 80) und der Vakuummembran (4) einer Rotationsbewegung der Motorachse (10) um einen Gesamtwinkel von 360° entspricht.

3. Membranvakuumpumpe nach einem der Ansprüche 1 bis 2, wobei der erste Rotationswinkel grösser als 180° ist, wobei er vorzugsweise 200° bis 300° beträgt und noch bevorzugter 240° bis 270° beträgt.

4. Membranvakuumpumpe nach einem der Ansprüche 1 bis 3, wobei der zweite Rotationswinkel kleiner als 180° ist, wobei er vorzugsweise 60° bis 160° beträgt und noch bevorzugter 90° bis 120° beträgt.

5. Membranvakuumpumpe nach einem der Ansprüche 1 bis 4, wobei das Drehmoment an der Motorachse (10) für die Rückwärtsbewegung des Verbindungselements (5, 80) über einen Winkel von grösser als 180° verteilt ist.

6. Membranvakuumpumpe nach einem der Ansprüche 1 bis 5, wobei das Getriebe ein Kurvenscheibengetriebe (6) ist, welches eine von einer Kreisform abweichende und im Betrieb der Membranvakuumpumpe rotierende Kurvenscheibe (60, 61) aufweist.

7. Membranvakuumpumpe nach Anspruch 6, wobei das Verbindungselement eine Verbindungsstange (5) ist, welche mit einem ersten Ende mit der Vakuummembran (4) verbunden ist und welche mit einem zweiten Ende an der Kurvenscheibe (60, 61) gelagert ist, und wobei eine Führung vorhanden ist, welche die Verbindungsstange (5) in die annähernd lineare, vorzugsweise lineare, Bewegung führt.

8. Membranvakuumpumpe nach einem der Ansprüche 6 oder 7, wobei mindestens die Kurvenscheibe (60, 61) aus Kunststoff besteht.

9. Membranvakuumpumpe nach einem der Ansprüche 1 bis 5, wobei das Getriebe ein Mehrgelenkgetriebe (7, 8), vorzugsweise ein Viergelenkgetriebe, ist.

10. Membranvakuumpumpe nach Anspruch 9, wobei das Verbindungselement (5, 80) ein Gelenkglied des Getriebes (7, 8) bildet.

11. Membranvakuumpumpe nach einem der Ansprüche 9 oder 10, wobei das Mehrgelenkgetriebe (7, 8) mehrere schwenkbar relativ zueinander gehaltene Gelenkglieder (73, 74, 5; 82, 83, 84, 80) aufweist.

12. Membranvakuumpumpe nach einem der Ansprüche 9 bis 11, wobei das Getriebe (7, 8) einstückig ausgebildet ist, wobei die Schwenkachsen durch Filmscharniere (86, 87, 88) gebildet sind.

13. Membranvakuumpumpe nach Anspruch 12, wobei das Getriebe (8) gemeinsam mit dem Verbindungselement (80) einstückig ausgebildet ist.

14. Membranvakuumpumpe nach einem der Ansprüche 9 bis 13, wobei das Getriebe (6, 7, 8) und/oder das Verbindungselement (5, 80) aus Kunststoff gefertigt sind.

## Claims

1. A diaphragm vacuum pump with a pump chamber (3), a vacuum diaphragm (4) for generating an underpressure in the pump chamber (3), an electric motor (1) with a motor shaft (10), a gear (6, 7. 8) and a connecting element (5, 80),
wherein the gear (6, 7, 8) converts a rotational movement of the motor shaft (10) into a cyclical, approximately linear forward and rearward movement of the connecting element (5, 80), as a result of which the connecting element (5, 80) effects a cyclical movement of the vacuum diaphragm (4),
wherein a second rotation angle of the motor shaft (10) effects the forward movement of the connecting element (5, 80) per cycle,
**characterized in that**
the rearward movement of the connecting element (5, 80) per cycle is effected by a first rotation angle of the motor shaft (10) which, on account of the gear (6, 7, 8), is not of the same magnitude as a second rotation angle of the motor shaft (10),
and that the rearward movement of the connecting element (5, 80) defines a pump stroke of the cycle that generates the underpressure in the pump chamber (3), and wherein the first rotation angle is greater than the second rotation angle.

2. The diaphragm vacuum pump according to claim 1, wherein a movement cycle of the connecting element (5, 80) and of the vacuum diaphragm (4) corresponds to a rotational movement of the motor shaft (10) about a total angle of 360°.

3. The diaphragm vacuum pump according to claim 1, wherein the first rotation angle is greater than 180°, preferably is 200° to 300°, more preferably is 240° to 270°.

4. The diaphragm vacuum pump according to one of the claims 1 to 3, wherein the second rotation angle is smaller than 180° preferably is 60° to 160°, more preferably is 90° to 120°.

5. The diaphragm vacuum pump according to one of the claims 1 to 4, wherein the torque on the motor shaft (10) for the rearward movement of the connecting element (5, 80) is distributed across an angle of greater than 180°.

6. The diaphragm vacuum pump according to one of the claims 1 to 5, wherein the gear is a cam disc gear (6) which has a cam disc (60, 61) that deviates from a circle shape and that rotates during the operation of the diaphragm vacuum pump.

7. The diaphragm vacuum pump according to claim 6, wherein the connecting element is a connecting rod (5) which with a first end is connected to the vacuum diaphragm (4) and which with a second end is mounted on the cam disc (60, 61), and wherein a guide means is present which guides the connecting rod (5) in the approximately linear, preferable linear movement.

8. The diaphragm vacuum pump according to claims 6 or 7, wherein at least the cam disc (60, 61) is made from plastic.

9. The diaphragm vacuum pump according to one of the claims 1 to 5, wherein the gear is a multi-bar linkage, preferably a a four-bar linkage.

10. The diaphragm vacuum pump according to claim 9, wherein the connecting element (5, 80) forms a link member of the gear (7, 8).

11. The diaphragm vacuum pump according to one of the claims 9 or 10, wherein the multi-bar linkage (7, 8) has several link members (73, 74, 5; 82, 84, 80) held pivotably relative to one another.

12. The diaphragm vacuum pump according to one of the claims 9 to 11, wherein the gear (7, 8) is designed in one piece, and wherein the pivot shafts are formed by film hinges (86, 87, 88).

13. The diaphragm vacuum pump according to claim 12, wherein the gear (8) is formed in one piece together with the connecting element (80).

14. The diaphragm vacuum pump according to one of the claims 9 to 13, wherein the gear (6, 7, 8) and/or the connecting element (5, 80) is made from plastic.

## Revendications

1. Pompe à vide à membrane ayant une chambre de pompage (3), une membrane à vide (4) pour générer une dépression dans la chambre de pompage (3), un moteur électrique (1) avec un axe de moteur (10), un engrenage (6, 7, 8) et un élément de liaison (5, 80),
l'engrenage (6, 7, 8) convertissant un mouvement de rotation de l'axe du moteur (10) en un mouvement cyclique vers l'avant et vers l'arrière, approximativement linéaire, de l'élément de liaison (5, 80), l'élément de liaison (5, 80) provoquant ainsi un mouvement cyclique de la membrane à vide (4),
un deuxième angle de rotation de l'axe du moteur (10) provoquant le mouvement vers l'avant de l'élément de liaison (5, 80) par cycle,
**caractérisé en ce que** le mouvement vers l'arrière de l'élément de liaison (5, 80) par cycle est provoqué par un premier angle de rotation de l'axe du moteur (10) qui, en raison de l'engrenage (6, 7, 8), n'est pas égal au deuxième angle de rotation de l'axe du moteur (10),
et **en ce que** le mouvement vers l'arrière de l'élément de liaison (5, 80) définit une course de pompage du cycle qui produit le vide dans la chambre à vide (3), et dans lequel le premier angle de rotation est plus grand que le deuxième angle de rotation.

2. Pompe à vide à membrane selon la revendication 1, dans laquelle un cycle de mouvement de l'élément de liaison (5, 80) et de la membrane à vide (4) correspond à un mouvement de rotation de l'axe du moteur (10) selon un angle total de 360°.

3. Pompe à vide à membrane selon l'une quelconque des revendications 1 à 2, dans laquelle le premier angle de rotation est supérieur à 180°, de préférence compris entre 200° et 300°, et de manière encore plus préférée compris entre 240° et 270°.

4. Pompe à vide à membrane selon l'une quelconque des revendications 1 à 3, dans laquelle le deuxième angle de rotation est inférieur à 180°, de préférence compris entre 60° et 160°, et de manière encore plus préférée compris entre 90° et 120°.

5. Pompe à vide à membrane selon l'une des revendications 1 à 4, dans laquelle le couple sur l'axe du moteur (10) pour le mouvement vers l'arrière de l'élément de liaison (5, 80) est réparti sur un angle supérieur à 180°.

6. Pompe à vide à membrane selon l'une des revendications 1 à 5, dans laquelle l'engrenage est un engrenage à came (6) qui présente une came (60, 61) de forme différente d'un cercle et qui tourne pendant le fonctionnement de la pompe à vide à membrane.

7. Pompe à vide à membrane selon la revendication 6, dans laquelle l'élément de liaison est une tige de liaison (5) qui est reliée par une première extrémité à la membrane à vide (4) et qui est montée par une deuxième extrémité sur la came (60, 61), et dans laquelle il existe un guidage qui guide la tige de liaison (5) dans le mouvement approximativement linéaire, de préférence linéaire.

8. Pompe à vide à membrane selon l'une des revendications 6 ou 7, dans laquelle au moins la came (60, 61) est en matière plastique.

9. Pompe à vide à membrane selon l'une quelconque des revendications 1 à 5, dans laquelle l'engrenage est un engrenage à articulations multiples (7, 8), de préférence un engrenage à quatre articulations.

10. Pompe à vide à membrane selon la revendication 9, dans laquelle l'élément de liaison (5, 80) forme un élément d'articulation de l'engrenage (7, 8).

11. Pompe à vide à membrane selon l'une des revendications 9 ou 10, dans laquelle l'engrenage à articulations multiples (7, 8) comprend plusieurs éléments d'articulation (73, 74, 5 ; 82, 83, 84, 80) maintenus pivotants les uns par rapport aux autres.

12. Pompe à vide à membrane selon l'une quelconque des revendications 9 à 11, dans laquelle l'engrenage (7, 8) est formé d'un seul tenant, les axes de pivotement étant formés par des charnières à film (86, 87, 88).

13. Pompe à vide à membrane selon la revendication 12, dans laquelle l'engrenage (8) est réalisé d'un seul tenant avec l'élément de liaison (80).

14. Pompe à vide à membrane selon l'une quelconque des revendications 9 à 13, dans laquelle l'engrenage (6, 7, 8) et/ou l'élément de liaison (5, 80) sont fabriqués en matière plastique.
